# EUROPEAN PATENT APPLICATION

(11) **EP 1 944 049 A1**
(43) Date of publication of application: **16.07.2008**
(21) Application number: 06822675.2
(22) Date of filing: 31.10.2006
(51) Int. Cl.: A61M 5/158

(54) **NEEDLE-STENT DEVICE**

(30) Priority: 01.11.2005 JP 2005318884
(71) Applicant: TERUMO KABUSHIKI KAISHA, Tokyo 151-0072 (JP)
(72) Inventor: OGAWA, Junichi, Yamanashi 409-3853 (JP); YAMAMORI, Yoshiki, Somerset, New Jersey 08873 (US); MURASHITA, Takato, Yamanashi 409-3853 (JP)
(74) Representative: TBK-Patent
(86) International application number: PCT/JP2006/321746
(87) International publication number: WO 2007/052655

(57) **Abstract**

A needle-stent device includes an inner needle having a sharp needlepoint at a tip thereof, an inner needle hub fixed to an inner needle base, a hollow outer needle into which the inner needle is inserted, an outer needle hub fixed to the outer needle base, a tube connected to the base of the outer needle hub so that an inner cavity thereof communicates with an inner cavity of the outer needle, an opening formed at the base or the side of the outer needle so as to communicate with the inner cavity of the outer needle, a handling part, which is formed in the outer needle and by which the inner and outer needles are moved in a longitudinal direction thereof when the inner needle is inserted into the outer needle, and a locking means which is switchable, when the inner needle is inserted into the outer needle, between a locked state (i.e., the inner needle hub being locked to the outer needle hub) and an unlocked state (i.e., the locked state being released).

## Description

### TECHNICAL FIELD

The present invention relates to a needle-stent device, which is made to puncture a blood vessel and indwell within the blood vessel at a time of, for example, carrying out an infusion.

### BACKGROUND ART

In the case of performing an infusion on a patient, or upon similar occasions, an indwelling needle connected to an infusion line is caused to puncture a blood vessel and indwell within the blood vessel.

Such an indwelling needle includes a hollow outer needle, an outer needle hub fixed to a base (proximal end) of the outer needle, an inner needle having a sharp needlepoint at a tip (distal end) thereof and which is inserted into the outer needle, and an inner needle hub fixed to the base of the inner needle (refer to, for example, Japanese Laid-Open Patent Publication No. 10-179734).

When the indwelling needle is made to puncture a patient's blood vessel, the inner needle is inserted into the outer needle, the needlepoint of the inner needle protrudes from the tip of the outer needle, and in such an assembled condition (needle-stent device condition), a puncturing operation is carried out. In the assembled condition, normally, an infusion line connector is connected to the outer needle hub.

Then, when the needlepoint of the inner needle has reached the inside of the blood vessel, blood flowing through the opening at the needlepoint passes through the inner cavity of the inner needle, and flows into the inside of the transparent inner needle (flashback). This makes it possible to confirm (visually check) that the blood vessel has been accessed by the inner needle.

After confirmation of flashback, the inner needle and the outer needle are slightly advanced to insert the outer needle into the blood vessel (to achieve puncture).

Next, while gripping the outer needle by hand, the inner needle is pulled out of the outer needle. Then, an infusion liquid is infused through the connected infusion line and the outer needle.

Meanwhile, the outer needle hub is provided with vanes forming an operating part thereof. When the inner needle and the outer needle are made to puncture the living body, the needle-stent device is placed in the assembled condition, and a puncturing operation is carried out on the skin while pinching the vanes with the fingers.

However, the needle-stent device described in the aforementioned patent document has a problem in that, at the time of carrying out the puncturing operation, the inner needle may be pushed by the skin before the skin is punctured, so as to retract into the outer needle. That is, operability (manipulatability) in performing the puncturing operation may be poor.

### DISCLOSURE OF INVENTION

It is an object of the present invention to provide a needle-stent device which is excellent in operability at the time of carrying out a puncturing operation.

In order to attain the above object, according to the present invention, there is provided a needle-stent device including:
an inner needle having a sharp needlepoint at a tip thereof;
an inner needle hub fixed to a basal part of the inner needle;
a hollow outer needle into which the inner needle is inserted;
an outer needle hub fixed to a basal part of the outer needle;
a tube connected to a basal part of the outer needle hub so that an inner cavity thereof communicates with an inner cavity of the outer needle;
an opening formed at a basal part or a side part of the outer needle hub so as to communicate with the inner cavity of the outer needle;
a handling part, which is provided in the outer needle hub and by which the inner needle and the outer needle are moved in a longitudinal direction thereof in an inserted condition in which the inner needle is inserted into the outer needle; and
a locking means which is switchable, in the inserted condition, between a locked state in which the inner needle hub is locked to the outer needle hub and an unlocked state in which the locked state is released.

According to the present invention, as set forth above, the needle-stent device is excellent in operability at the time of carrying out a puncturing operation.

In addition, in the needle-stent device according to the present invention, preferably, a tube is connected to the opening.

This makes it possible to supply a liquid, such as a liquid medicine, into the outer needle through the tube.

Further, in the needle-stent device according to the present invention, preferably, the tube is inserted in the inner needle hub.

This ensures that the tube can be prevented from acting as an obstacle when operations are carried out by the needle-stent device.

Preferably, in the needle-stent device according to the present invention,
the locking means has a projecting part disposed on one of the inner needle hub and the outer needle hub, and an engaging part disposed on the other of the inner needle hub and the outer needle hub, which is capable of engagement with the projecting part, and
the engaging part engages with the projecting part in the locked state, while the engaging part is disengaged from the projecting part in the unlocked state.

This ensures that, when the handling part is operated to carry out a puncturing operation in the locked state, the inner needle hub is in a state of being fixed to the outer needle hub. Therefore, a problem wherein the inner needle is pushed by the part to be punctured (objective part) and retracted into the outer needle before the objective part is punctured is prevented or restrained, so that the inner needle can puncture the objective part reliably. In other words, the needle-stent device is excellent in operability when the puncturing operation is carried out. In addition, after puncturing, an unlocked state can be obtained, and, in such an unlocked condition, the inner needle can be pulled out of the outer needle.

Further, in the needle-stent device according to the present invention, preferably, the engaging part is rotatably supported, and is turned between the locked state and the unlocked state.

This makes it possible to switch from the locked state to the unlocked state through a simple operation.

In addition, in the needle-stent device according to the present invention, preferably, the engaging part is disposed on the outer needle hub and serves as the handling part in the unlocked state.

This renders the needle-stent device superior at the time the puncturing operation is performed.

Further, in the needle-stent device according to the present invention, preferably, the engaging part is disposed on the outer needle hub, and is switchable not only between the locked state and the unlocked state, but also is switchable to a retracted state, in which at least a portion of the engaging part is retracted from an outer peripheral portion of the outer needle hub.

This ensures that, when the outer needle, from which the inner needle has been pulled off, is left indwelling with a pressure sensitive adhesive tape applied thereto, for example, the pressure sensitive adhesive tape can be securely prevented from becoming caught on the engaging part. Therefore, the pressure sensitive adhesive tape can be prevented reliably from becoming broken.

In addition, in the needle-stent device according to the present invention, preferably, the retracted state is a stored state in which at least a portion of the engaging part is stored in the outer needle hub.

This ensures that, when the outer needle, from which the inner needle has been pulled off, is left indwelling with a pressure sensitive adhesive tape applied thereto, for example, the pressure sensitive adhesive tape is securely prevented from becoming caught on the engaging part. Therefore, the pressure sensitive adhesive tape can be prevented reliably from becoming broken.

Further, in the needle-stent device according to the present invention, preferably, the projecting part is composed of a plurality of claw parts provided along the outer periphery of the inner needle hub, wherein a plurality of engaging parts are provided corresponding respectively to the claw parts.

This ensures that, when the operating part is operated to perform a puncturing operation in the locked state, the inner needle hub remains in a state of being fixed to the outer needle hub. Therefore, a problem wherein the inner needle is pushed by the objective part and retracted into the outer needle before the objective part is punctured is prevented or restrained, so that the inner needle can puncture the objective part reliably. In other words, the needle-stent device is excellent in operability at the time of carrying out a puncturing operation. In addition, after completion of puncturing, an unlocked state can be obtained, such that, in the unlocked condition, the inner needle can be pulled out of the outer needle.

In addition, the needle-stent device according to the present invention, preferably, further includes a claw part operating member by which the claw parts are moved toward and away from each other.

This makes it possible to easily displace each of the claw parts.

Further, in the needle-stent device according to the present invention, preferably, respective claw part operating members are provided at each of the claw parts, and the claw parts are spaced away from each other when the claw part operating members are moved toward each other.

This ensures that the claw parts, having been engaged in the locked state, are disengaged respectively from the engaging parts, so that the needle-stent device can be placed in the unlocked state.

In addition, the needle-stent device according to the present invention, preferably, further includes an urging part for urging the claw part operating member in a direction such that the claw parts are moved toward each other.

This ensures that the needle-stent device is reliably placed in the locked state. In other words, a problem in which the needle-stent device is unintentionally placed in an unlocked state, with the result that the inner needle hub becomes released from the outer needle hub, can be securely prevented.

Further, in the needle-stent device according to the present invention, preferably, the claw part is provided on the inner needle hub, and the engaging part is provided on the outer needle hub.

This enhances operability of the needle-stent device.

### BRIEF DESCRIPTION OF THE DRAWINGS

FIG. 1 is a perspective view of a first embodiment of the needle-stent device according to the present invention;
FIG. 2 is a sectional view taken along line A-A of FIG. 1;
FIG. 3 is a sectional view taken along line A-A of FIG. 1;
FIG. 4 is a sectional view taken along line A-A of FIG. 1;
FIG. 5 is a sectional view taken along line B-B of FIG. 1:
FIG. 6 is a sectional view taken along line B-B of FIG. 1:
FIG. 7 is a sectional view taken along line B-B of FIG. 1;
FIG. 8 is a sectional view taken along line B-B of FIG. 1;
FIG. 9 is a perspective view, corresponding to FIG. 8, of the needle-stent device shown in FIG. 1:
FIG. 10 is a perspective view of the needle-stent device shown in FIG. 1, in a condition in which a tube has been detached from an inner needle hub;
FIG. 11 is a perspective view of a seal member possessed by the needle-stent device shown in FIG. 1;
FIG. 12 is a partial longitudinal sectional view of a needle-stent device according to the present invention (second embodiment);
FIG. 13 is a partial longitudinal sectional view of the needle-stent device according to the present invention (second embodiment); and
FIG. 14 is a partial longitudinal sectional view of an inner needle hub possessed by a needle-stent device according to the present invention (third embodiment).

### BEST MODE FOR CARRYING OUT THE INVENTION

The needle-stent device according to the present invention will be described in detail below, based on preferred embodiments shown in the accompanying drawings.

### <First Embodiment>

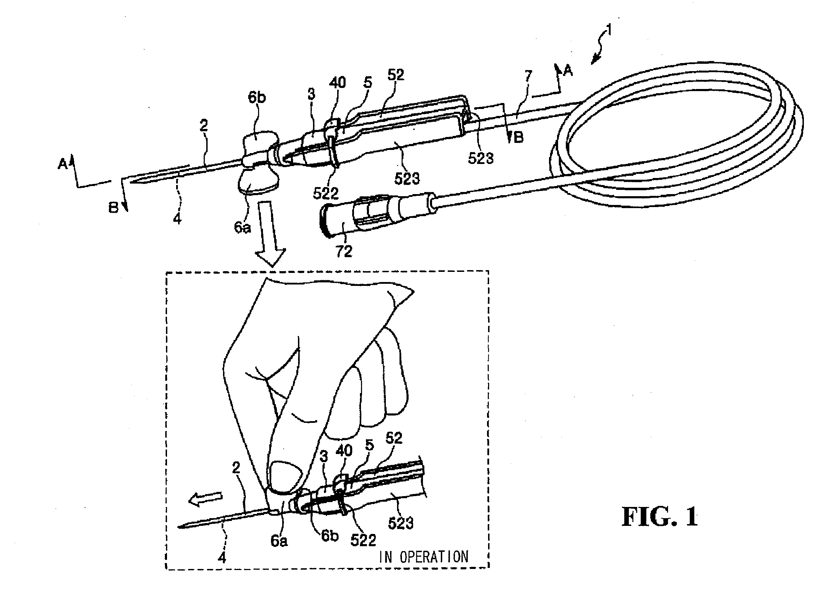

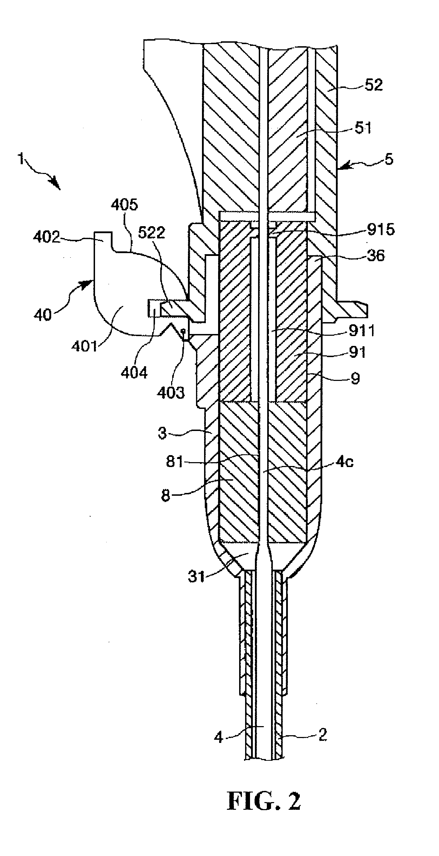

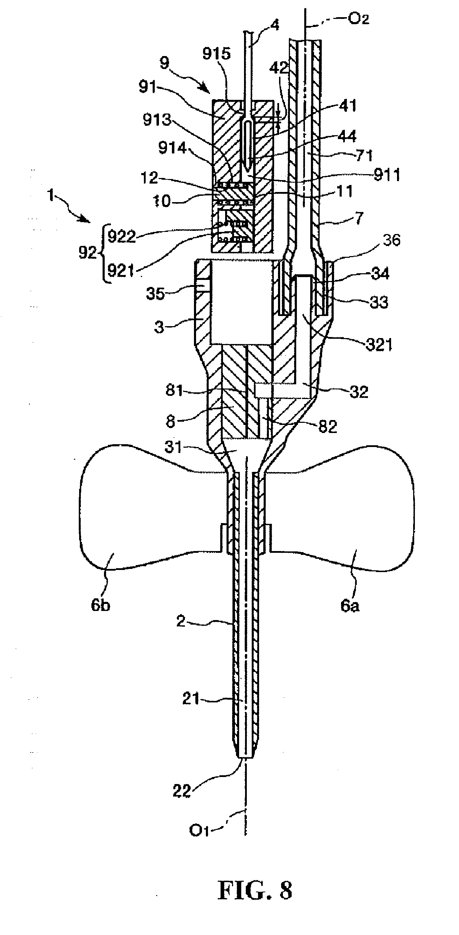

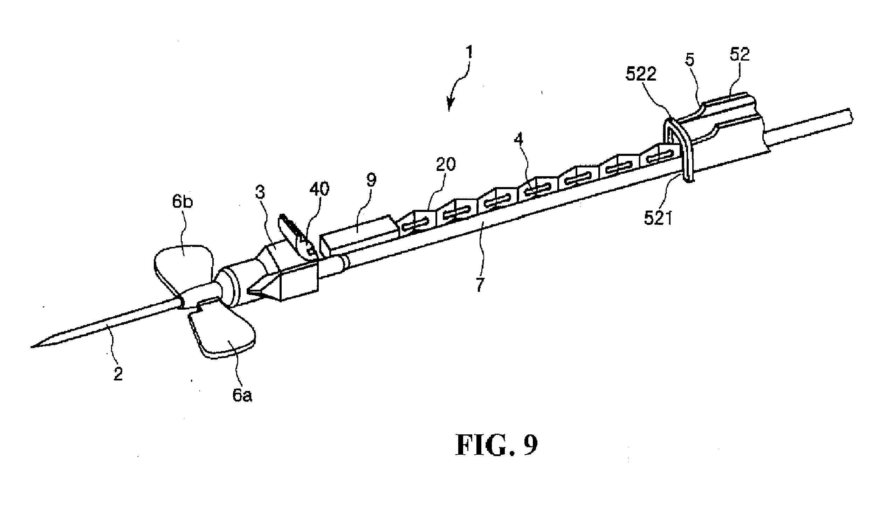

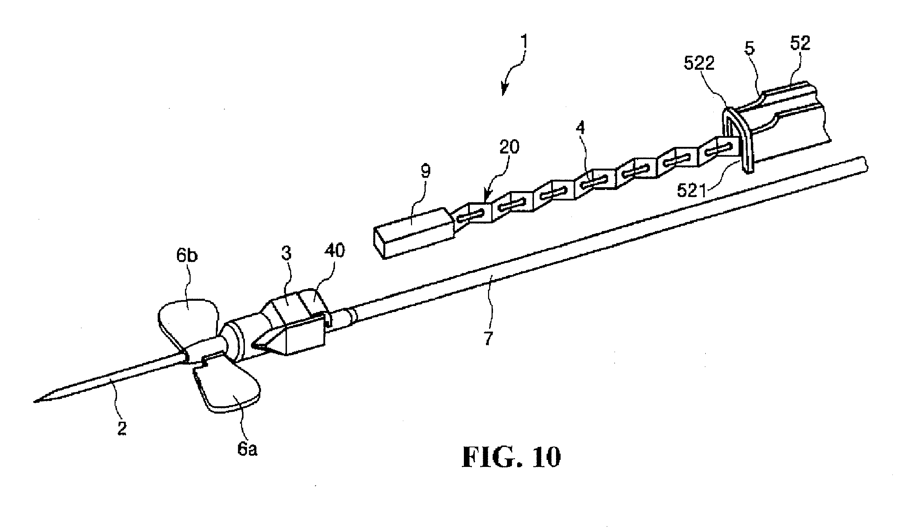

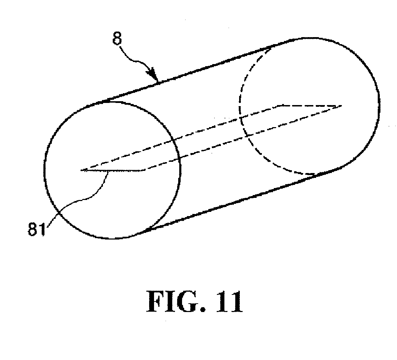

FIG. 1 is a perspective view of a first embodiment of the needle-stent device; FIGS. 2 to 4 are sectional views taken along line A-A of FIG. 1; FIGS. 5 to 8 are sectional views taken along line B-B of FIG. 1; FIG. 9 is a perspective view, corresponding to FIG. 8, of the needle-stent device shown in FIG. 1; FIG. 10 is a perspective view of the needle-stent device shown in FIG. 1, in a condition in which a tube has been detached from an inner needle hub; and FIG. 11 is a perspective view of a seal member possessed by the needle-stent device shown in FIG. 1.

Incidentally, in the following descriptions, the right side in FIGS. 1, 9 and 10 shall be referred to as "the base", and the left side as "the tip". Further, the upper side in FIGS. 2 to 8 shall be referred to as "the base", and the lower side as "the tip". In addition, in FIGS. 2 to 8, a slip-off preventive means, which is possessed by the needle-stent device according to the present invention, is omitted from illustration.

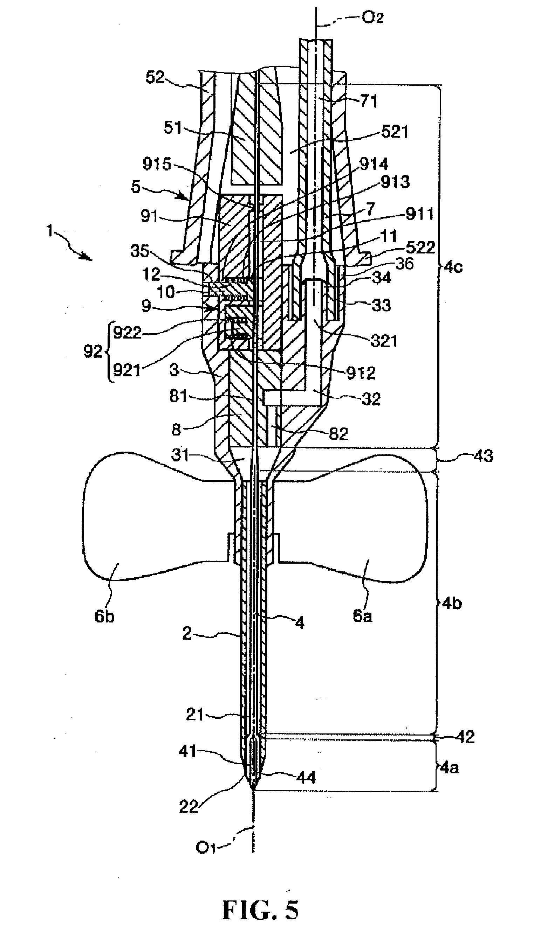

The needle-stent device 1 shown in FIG. 1 (as well as in FIGS. 5 to 10) includes a hollow outer needle 2, an outer needle hub 3 fixed to a basal part of the outer needle 2, an inner needle 4 inserted into the outer needle 2, an inner needle hub 5 fixed to a basal part of the inner needle 4, and a tube 7 connected to a basal part 36 (or a side part) of the outer needle hub 3, so that an inner cavity 71 thereof communicates with the inner cavity 21 of the outer needle 2. Configurations of these components and sections shall be described below.

A needle having a certain degree of flexibility is used as the outer needle 2. The material constituting the outer needle 2 preferably is a resin material, particularly a soft resin material. Specific examples of the material include fluoro-resins such as PTFE, ETFE, PFA, olefin resins such as polyethylene, polypropylene, and mixtures thereof, polyurethane, polyester, polyamide, polyether nylon resins, and mixtures of the olefin resin with an ethylene-vinyl acetate copolymer, etc.

The entirety or a portion of the outer needle 2 may have a property such that the inside thereof can be visually confirmed. In addition, the material constituting the outer needle 2 may be admixed with a radiopaque agent, such as barium sulfate, barium carbonate, bismuth carbonate, tungstic acid, etc., to obtain a contrast property.

The outer needle hub 3 is secured (fixed) in a liquid-tight manner to a basal part of the outer needle 2 by a method such as caulking, fusing (heat fusing, high-frequency fusing, or the like), bonding with an adhesive, etc.

The outer needle hub 3 is composed of a substantially tubular member, the inside 31 of which communicates with the inner cavity 21 of the outer needle 2.

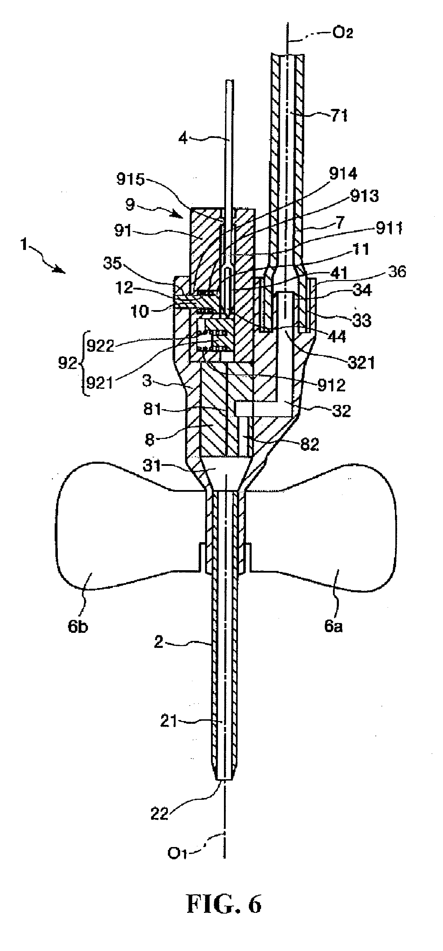

A wall portion on the right side in FIG. 5 (as well as in FIGS. 6 to 8) of the outer needle hub 3 is provided with a conduit 32, which opens at one end thereof into the inside 31 of the outer needle hub 3. The conduit 32 is substantially L-shaped, wherein the other end of the conduit 32 opens into a recessed part 33, which is formed in the base of the outer needle hub 3, thereby forming an opening 321. In addition, at the tip face (bottom face) of the recessed part 33, a projecting part (connecting part) 34 having an annular shape surrounding the opening 321 is formed so as to project in the basal direction (proximal direction).

The projecting part 34 is inserted into the inner cavity 71 of a tip part of the tube 7, and one end part (tip part) of the tube 7 is connected to the outer needle hub 3. This makes it possible to supply a liquid, such as a liquid medicine, into the outer needle 2 (outer needle hub 3) through the tube 7.

In addition, on the left and right sides in FIG. 5 (as well as in FIGS. 6 to 8) of the outer needle hub 3, a pair of vanes 6a and 6b are formed integrally with the outer needle hub 3, thereby forming a handling part. The vanes 6a and 6b are flexible, and are configured so that the vanes 6a, 6b can be opened and closed by bending or curving in the vicinity of joint portions between the vanes 6a, 6b and the outer needle hub 3.

When the outer needle 2 and the inner needle 4 are made to puncture a blood vessel or the like, the vanes 6a and 6b are pinched with the fingers to bring the vanes 6a, 6b into a closed condition, whereby movement of the inner needle 4 and the outer needle 2 along the longitudinal direction can be facilitated, i.e., the puncturing operation can be conducted (see FIG. 1). When the outer needle 2 is made to indwell, the outer needle hub 3 (see FIG. 10), with the vanes 6a and 6b in an opened condition, is fixed to the skin using a pressure sensitive adhesive tape or the like.

The inner needle 4 having a sharp needlepoint 41 at the tip thereof is inserted into the outer needle 2. The needle-stent device 1 is used in a condition in which the inner needle 4 is inserted into the outer needle 2 and wherein the inner needle hub 5 is fitted into the outer needle hub 3, i.e., the condition shown in FIGS. 1, 2 and 5. Hereinafter, this condition will be referred to as "the assembled condition".

The length of the inner needle 4 is set at a level such that at least the needlepoint 41 thereof protrudes from the tip opening 22 of the outer needle 2 in the assembled condition.

The inner needle 4 may be a hollow needle, but preferably, the inner needle 4 is a solid needle. When the inner needle 4 is formed as a solid needle, sufficient strength can be secured, while the outer diameter of the inner needle 4 is set to be small. Additionally, when the inner needle 4 is solid, any danger of blood remaining inside or flowing out from the inner needle 4, when the inner needle 4 is discarded after completion of a procedure, can be prevented. Thus, high safety is ensured.

Incidentally, in the case that the inner needle 4 is a hollow needle, blood flows into the hollow section of the inner needle 4 when the inner needle 4 punctures a blood vessel, so that flashback of the blood can be confirmed. On the other hand, when the inner needle 4 is solid, blood flows into a gap between the inner needle 4 and the outer needle 2, whereby flashback of the blood can be confirmed more swiftly.

Incidentally, the inner needle 4 can be made with a configuration including both a hollow section and a solid section (for example, a configuration in which a portion of the inner cavity of a hollow needle is filled, so as to obtain a hollow section on the tip side and a solid section on the base side). However, when the entire body of the inner needle 4 is composed of a single member, a reduction in the cost of the inner needle 4 can be achieved.

In addition, the inner needle 4 has a plurality of sections (three, in the present embodiment), which differ in outer diameter. Specifically, the inner needle 4 has a maximum outer diameter section 4a on the tip side (tip part) thereof, a minimum outer diameter section 4c on the base side thereof, and an intermediate outer diameter section 4b provided between the maximum outer diameter section 4a and the minimum outer diameter section 4c.

Further, the inner needle 4 is provided with a first varied outer diameter section 42 (which is continuously varied in outer diameter) at the boundary between the maximum outer diameter section 4a and the intermediate outer diameter section 4b, and with a second varied outer diameter section 43 (which is continuously varied in outer diameter) between the intermediate outer diameter section 4b and the minimum outer diameter section 4c.

At each of the varied outer diameter sections 42 and 43, the outer diameter of the inner needle 4 may be varied stepwise. However, when the inner needle 4 is continuously varied in outer diameter (i.e., tapered), each of the varied outer diameter sections 42 and 43 can be prevented from becoming caught on a tip edge part of the slit 81 in the seal member 8 (described later), or on a tip edge part of a inner needle passage 911 within a protector body 91, or the like, when the inner needle 4 is withdrawn from the outer needle 2. Therefore, the operation of withdrawing the inner needle 4 from the outer needle 2 can be carried out smoothly and assuredly.

Incidentally, the varied outer diameter sections 42 and 43 may be formed at the time of producing the inner needle 4. Alternatively, a step or steps inevitably carried out at the time of forming a groove 44 (described later) may also be utilized.

In addition, the maximum outer diameter section 4a has an outer diameter set approximately equal to the inner diameter of the outer needle 2, so that the maximum outer diameter section 4a makes close contact with the inside surface of the outer needle 2 in a condition in which the inner needle 4 is inserted into the outer needle 2. An outer peripheral part of the maximum outer diameter section 4a is provided with a recessed groove (conduit) 44 therein formed along the longitudinal direction of the inner needle 4. The groove 44 provides communication between the tip opening 22 of the outer needle 2 and the inside 31 of the inner needle hub 3, in a condition in which the inner needle 4 is inserted into the outer needle 2. The groove 44 functions as a conduit for blood (body fluid), at the time of puncturing a blood vessel, for example. This makes it possible to securely confirm flashback of the blood.

Examples of materials constituting the inner needle 4 as described above may include metallic materials, such as stainless steel, aluminum, aluminum alloys, titanium, titanium alloys, etc.

As shown in FIGS. 2 and 5, the inner needle hub 5 is secured (fixed) to a basal part of the inner needle 4. The inner needle hub 5 is composed of a fixing section 51 for fixing the inner needle 4, and a cover section 52 provided on the outer peripheral side of the fixing section 51. Preferably, the fixing section 51 and the cover section 52 are formed integrally.

In addition, in the assembled condition, the tube 7 is disposed between the fixing section 51 and the cover section 52. Namely, in the assembled condition, the tube 7 is inserted into the inner needle hub 5. This ensures that the tube 7 can be prevented from obstructing operations of the needle-stent device 1.

In addition, the cover section 52 is provided with a pair of guides 523, 523 for guiding the tube 7 (see FIG. 1). The guides 523 constitute side walls (side parts) of the cover section 52, and serve to guide the tube 7 so that the center axis O₂ of the tube 7 at the tip part thereof will be substantially parallel to the longitudinal direction of the inner needle hub 5 (the center axis O₁ of the outer needle 2).

In addition, when the inner needle 4 is withdrawn from the outer needle 2, the tube 7 can be detached from the inner needle hub 5 through a clearance (gap 521) formed between both of the guides 523 (see FIGS. 9 and 10).

Fixing of the inner needle 4 to the inner needle hub 5 (fixing section 51) may be carried out by a method such as fitting, caulking, fusing, bonding with an adhesive, etc., or by a combination of these methods. Further, in the case that the inner needle 4 is hollow, it is necessary to provide a seal therein, for example, so that blood flowing back upon puncturing a blood vessel with the inner needle 4 will not run out through the base of the inner needle 4.

In addition, as shown in FIG. 1, a flange (projecting part) 522 is provided at the outer periphery of the tip of the inner needle hub 5. With the flange 522, for example, at the time the inner needle 4 is withdrawn from the outer needle 2, the fingers may be placed on the flange 522 to thereby perform the withdrawing operation, whereby operations can be carried out more easily and assuredly. Further, as shown in FIG. 2, the flange 522 engages with a tab 40 (described later) in the assembled condition.

The inner needle hub 5 and the above-described outer needle hub 3 are preferably formed from a transparent (colorless transparent), colored transparent or semitransparent resin, so as to have a property in which the inside thereof can be visually confirmed. This ensures that, when the outer needle 2 accesses a blood vessel, flashback of the blood flowing in through the groove 44 of the inner needle 4, can be visually confirmed, as mentioned previously. In addition, when the inner needle 4 is solid, all of the blood that is flashed back under the pressure inside the blood vessel, for example, flows back through the groove 44, which enhances visual confirmation thereof.

The materials constituting the outer needle hub 3, the inner needle hub 5 and the vanes 6a and 6b are not particularly limited. Examples of such materials include various resin materials such as polyolefins including polyethylene, polypropylene, ethylene-vinyl acetate copolymer, etc., polyurethane, polyamides, polyesters, polycarbonate, polybutadiene, polyvinyl chloride, etc.

The tube 7 is flexible and, as mentioned above, has one end thereof connected to the basal part 36 of the outer needle hub 3. A connector 72 is mounted (see FIG. 1) to the other end part (basal part) of the tube 7. A connector mounted to an end of an infusion line for supplying an infusion liquid (liquid medicine) to be dosed, or a mouth (tip part) of a syringe in which a liquid medicine is contained, is connected to the connector 72, for example.

Incidentally, the material constituting the tube 7 is not particularly limited. Examples of such materials include polyolefins such as polyethylene, polypropylene, ethylene-vinyl acetate copolymer, etc., polyvinyl chloride, polybutadiene, polyamides, polyesters, etc., among which polybutadiene is particularly preferred. When the tube 7 is formed from polybutadiene, the tube 7 has appropriate flexibility and chemical resistance, with an excellent property for preventing adsorption of medicines thereon.

Further, the needle-stent device 1 includes a cylindrical (block-like) seal member 8 at the inside 31 of the outer needle hub 3 (see FIG. 11). The seal member 8 is provided substantially in the center thereof with a slit 81, which penetrates the seal member 8 along the longitudinal direction.

As shown in FIG. 11, the slit 81 is in the shape of a straight line segment.

The seal member 8 in the assembled condition has a self-closing property such that, when the inner needle 4 inserted into the slit 81 is withdrawn, the slit 81 is self-closed by an elastic force of the seal member 8. This ensures that when the inner needle 4 is withdrawn, leakage of the liquid from the base of the outer needle hub 3 can be prevented, and asepsis inside of the outer needle hub 3 can be maintained.

In addition, as shown in FIGS. 2, 3 and 5, in the assembled condition, the minimum outer diameter section 4c of the inner needle 4 is located in the slit 81. This configuration prevents the seal member 8 (slit 81) from undergoing a permanent or semi-permanent deformation, which if it occurred, would lower the sealing performance.

Examples of the materials constituting the seal member 8 include various elastic materials, such as various rubber materials (particularly vulcanized rubbers) such as natural rubber, isoprene rubber, butyl rubber, butadiene rubber, styrene-butadiene rubber, urethane rubber, nitrile rubber, acrylic rubber, fluoro-rubber, silicone rubber, etc., various thermoplastic elastomers based on urethane, polyester, polyamide, olefin, styrene, etc., and mixtures thereof.

Further, the seal member 8 is provided with a conduit 82 at a location different from that of the slit 81. The conduit 82 opens on the tip face and on a side surface of the seal member 8, and is roughly L-shaped.

When the seal member 8 is inserted into the outer needle hub 3, and an opening where the conduit 82 opens on the side surface of the seal member 8 and an opening where the above-mentioned conduit 32 opens into the inside 31 of the outer needle hub 3 are set to coincide with each other, a crank-shaped junction conduit is formed (completed). The junction conduit provides communication between the inner cavity 21 of the outer needle 2 and the inner cavity 71 of the tube 7. Such a configuration makes it possible for the junction conduit to be comparatively short, and to prevent the outer needle hub 3 from becoming enlarged in size.

In the needle-stent device 1, the outer needle hub 3 is provided with a tab 40.

As shown in FIG. 2, the tab 40 includes a tab body 401, which has a columnar form with a substantially elliptic sectional shape, a lever (finger hook part) 402, and a turning shaft 403.

The tab body 401 is provided at a side surface thereof with a recessed part (engaging part) 404.

In addition, the ellipsoidal outer peripheral surface of the tab body 401 is provided with the lever 402 that projects in a tangential direction (the basal direction in FIG. 2). This ensures that the tab body 401 can be easily turned about the turning shaft 403 by placing a finger onto the lever 402.

Further, the turning shaft 403 comprises a shaft for supporting the tab body 401 (recessed part 404), so that the tab body 401 can be turned relative to the outer needle hub 3.

As shown in FIG. 2, in the assembled condition, the tab 40 (recessed part 404) can engage with the flange 522. This places the needle-stent device 1 in a locked state, in which the inner needle hub 5 is locked to the outer needle hub 3.

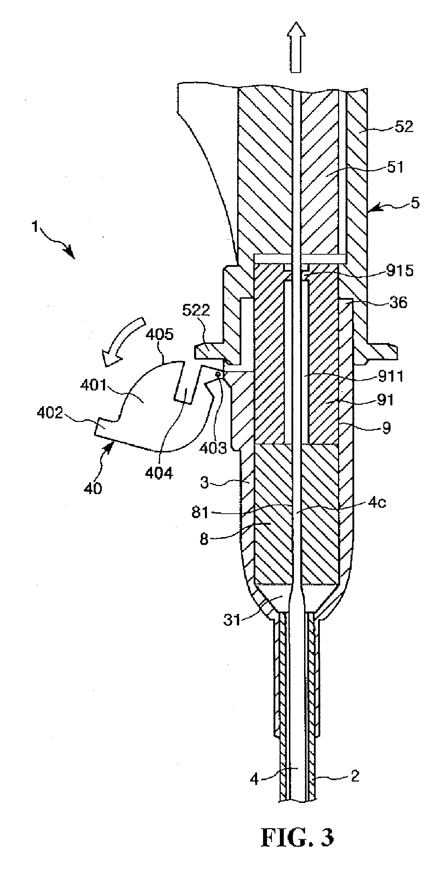

In addition, as shown in FIG. 3, when the tab 40 in the locked condition (engaged condition) is turned counterclockwise as shown in FIG. 3, the tab 40 is released from the flange 522. This places the needle-stent device 1 in an unlocked state, in which locking thereof is released.

With the tab 40 turned in this manner, the needle-stent device 1 is placed in the locked state, or alternatively, is placed in the unlocked state.

When, in the locked state, the needle-stent device 1 performs a puncturing operation by pinching the vanes 6a and 6b with the fingertips (see FIG. 1), the inner needle hub 5 is in a state of being fixed to the outer needle hub 3. Therefore, a problem in which the inner needle 4 is pushed by the skin and retracted into the outer needle 2 before the skin (blood vessel) is punctured can be prevented or restrained, so that the inner needle 4 punctures the skin without fail. In other words, the needle-stent device 1 is excellent in operability at the time of carrying out the puncturing operation.

Further, by turning the tab 40 after puncturing, the needle-stent device 1 can be placed in an unlocked state through an easy operation, so that the inner needle 4 can be pulled out from the outer needle 2. Then, the outer needle 2 with the inner needle 4 withdrawn therefrom (see FIG. 10) can be left to indwell inside the blood vessel.

In this case, by pressing (pushing) a pressing surface (basal surface) 405 of the tab 40, while in the unlocked state, with a finger while gripping the inner needle hub 5, the outer needle 2 and the outer needle hub 3 can be advanced into the blood vessel with one hand. Thus, the pressing surface 405 of the tab 40 performs a similar function to that of the vanes 6a, 6b.

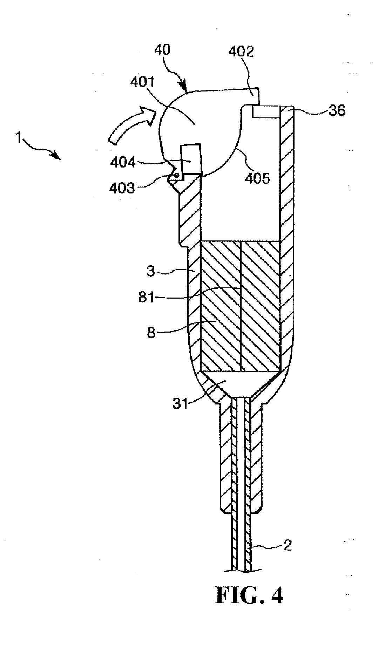

As shown in FIG. 4, in the needle-stent device 1, the tab 40 can be switched not only between the locked state (see FIG. 2) and the unlocked state (see FIG. 3), but also may be switched into a stored state (retracted state).

The stored state is a state obtained by turning the tab 40 (being in the unlocked state) clockwise as shown in FIG. 4, in which the majority of the tab 40 is retracted from the outer peripheral part of the outer needle hub 3. Specifically, in the stored state, the majority of the tab 40 is stored in the inside 31 of the outer needle hub 3.

With the tab 40 stored, i.e., when the tab 40 does not project from the outer peripheral part of the outer needle hub 3, a trouble wherein a pressure sensitive adhesive tape becomes caught by the tab 40 when the outer needle 2 is made to indwell can be securely prevented. Thus, the pressure sensitive adhesive tape can be reliably prevented from becoming broken. This ensures that the outer needle hub 3, with the vanes 6a and 6b in an opened state (see FIG. 10), can be securely fixed to the skin by use of the pressure sensitive adhesive tape.

In addition, as shown in FIG. 4, in the stored state, the lever 402 makes contact with a basal part 36 of the outer needle hub 3. This prevents the tab 40 from entering excessively into the inside 31 of the outer needle hub 3.

Thus, in the needle-stent device 1, the tab 40 and the flange 522 collectively constitute a locking means, by which the inner needle hub 5 is fixed to the outer needle hub 3 in the assembled condition.

Incidentally, the tab 40 is not limited to being provided on the outer needle hub 3, and may also be provided on the inner needle hub 5. In the event that the inner needle hub 5 is provided with the tab 40, the flange 522 that engages with the tab 40 must be provided on the side of the outer needle hub 3.

Further, although only one tab 40 has been provided in the configuration shown in the figures, the present invention is not limited to this configuration. For example, two or more tabs may be provided.

Furthermore, the needle-stent device 1 includes a protector 9 for covering at least the needlepoint 41 of the inner needle 4 when the inner needle 4 is withdrawn from the outer needle 2. The protector 9 will be described below as follows.

As shown in FIGS. 5 to 8, the protector 9 includes a protector body 91 having a substantially rectangular parallelepiped outer shape, and a shutter means 92 provided in the protector body 91.

Substantially in the center of the protector body 91, an inner needle passage 911, in which the inner needle 4 is inserted, is formed so as to penetrate through the protector body 91 along the longitudinal direction thereof.

The inner needle passage 911 is substantially circular in cross section, and the inner diameter thereof is equal to or slightly greater than the outer diameter of the maximum outer diameter section 4a of the inner needle 4.

In addition, the inside wall (the surface facing the inner needle passage 911) on the tip side of the protector body 91 is provided with a recessed part 912.

The shutter means 92 is stored in the recessed part 912. The shutter means 92 is composed of a block-shaped shutter member 921, and a coil spring (urging means) 922, which urges the shutter member 921 toward the side of the inner needle passage 911.

The shutter means 92 can be displaced between a first posture (the posture shown in FIG. 5), in which the majority of the shutter means 92 is retracted into the recessed part 912 and the inner needle 4 is insertable into the inner needle passage 911, and a second posture (the posture shown in FIG. 6) in which a portion of the shutter member 921 enters into the inner needle passage 911, so as to inhibit the needlepoint 41 of the inner needle 4 from passing therethrough.

With the above-described protector 9, the needlepoint 41 of the inner needle 4 can be speedily and safely covered by a simple operation after use. In addition, due to the action of the shutter means 92, once the needlepoint 41 has been covered, the needlepoint 41 is prevented from protruding from the tip of the protector 9 (the protector body 91). Therefore, an accident in which a worker erroneously punctures his or her finger or the like with the needlepoint 41 when discarding the inner needle 4, or on a similar occasion, can be prevented, so that high safety is thereby ensured.

Further, in the assembled condition, substantially the entirety of the protector 9 is covered with both the outer needle hub 3 and the inner needle hub 5. This ensures that the protector 9 does not obstruct a puncturing operation with the outer needle 2 and the inner needle 4, so that the puncturing operation can be performed more assuredly. Incidentally, the protector 9 may be substantially wholly covered, with either one of the outer needle hub 3 or the inner needle hub 5.

Furthermore, the protector 9 is configured so as to be located on the base side relative to the seal member 8 in the assembled condition. This eliminates the need to pass the protector 9 through the slit 81 in the seal member 8 when withdrawing the inner needle 4 from the outer needle 2. Therefore, the withdrawing operation can be performed more easily and securely. In addition, this configuration makes it possible for the overall length of the inner needle 4 to be shorter, whereby a reduction in the size of parts exclusive of the tube 7 of the needle-stent device 1 can be achieved.

As shown in FIGS. 9 and 10, the needle-stent device 1 has a connection member 20 that serves as a slip-off preventive means, for preventing the protector 9 from slipping off from the needlepoint 41 when the protector 9 covers the needlepoint 41.

The connection member 20 is configured to connect the protector 9 and the inner needle hub 5 to each other. This ensures that the protector 9 is securely prevented from slipping off from the inner needle hub 5 (needlepoint 41), so that a condition whereby the protector 9 covers the needlepoint 41 can be maintained securely. Therefore, an accident in which a worker or the like erroneously punctures his or her finger with the needlepoint 41 when discarding the inner needle 4, or on a similar occasion, is reliably prevented, so that high safety is ensured.

In addition, the connection member 20 has a bellows-like shape, and can therefore be extended and contracted as desired. The connection member 20 is contracted, or folded, in the assembled condition and becomes extended, or unfolded, in a condition wherein the inner needle 4 has been withdrawn from the outer needle 2 (the condition shown in FIGS. 9 and 10).

The aforementioned connection member 20 is contracted in the assembled condition, and is stored in a contracted state within the inner needle hub 5. This ensures that the connection member 20 does not form an obstacle during the puncturing operation, so that operability of the needle-stent device 1 can be enhanced. In addition, a merit results in that a reduction in size of the needle-stent device 1 can be achieved.

Further, in the condition in which the connection member 20 is contracted, as well as in the condition wherein the connection member 20 is extended, the inner needle 4 penetrates into the connection member 20. Thus, the inner needle 4 functions as a guide for the connection member 20 when the connection member 20 is extended and contracted. Therefore, the connection member 20 can be prevented from becoming contracted in an undesired manner, or from becoming contracted without being contained within the inner needle hub 5, when the needle-stent device 1 is placed in the assembled condition (during manufacturing thereof), for example.

In addition, in the assembled condition, the aforementioned needle-stent device 1 includes a fixing means for fixing the protector 9 to the outer needle hub 3, along with an engaging means (movement restraining means) for restraining movement of the inner needle 4 in a direction opposite to the needlepoint 41 in relation to the protector 9, as a result of engagement between the inner needle 4 and the protector 9 in a condition in which at least the needlepoint 41 of the inner needle 4 is covered by the protector 9. Next, the fixing means and the engaging means shall be described in greater detail below.

### <Fixing means>

First, the fixing means will be described.

The inside wall of the protector body 91 is provided with a through-hole 913 on the base side of the recessed part 912, and a projecting part 914, which projects toward the inside, is formed at the left end of the through-hole 913 as shown in FIG. 5.

A fixing pin 10, which has a flange part 11 on the right end thereof in FIG. 5, is inserted into the through-hole 913, in a condition in which a coil spring 12 is stored therein. In this condition, the left end of the coil spring 12 in FIG. 5 is in contact with the projecting part 914, and the right end in the figure is in contact with the flange part 11.

In addition, a through-hole 35 into which the fixing pin 10 can be inserted is formed in a basal part of the left side wall part of the outer needle hub 3, as shown in FIG. 5.

In a condition where the inner needle 4 is inserted into (penetrates) the inner needle passage 911, the right surface of the fixing pin 10 is in contact with the outer peripheral surface (outside surface 45) of the inner needle 4, while the left end part of the fixing pin 10 protrudes from the through-hole 913 and is inserted into the through-hole 35. By this arrangement, the protector 9 becomes fixed to the outer needle hub 3 (see FIGS. 5 and 6).

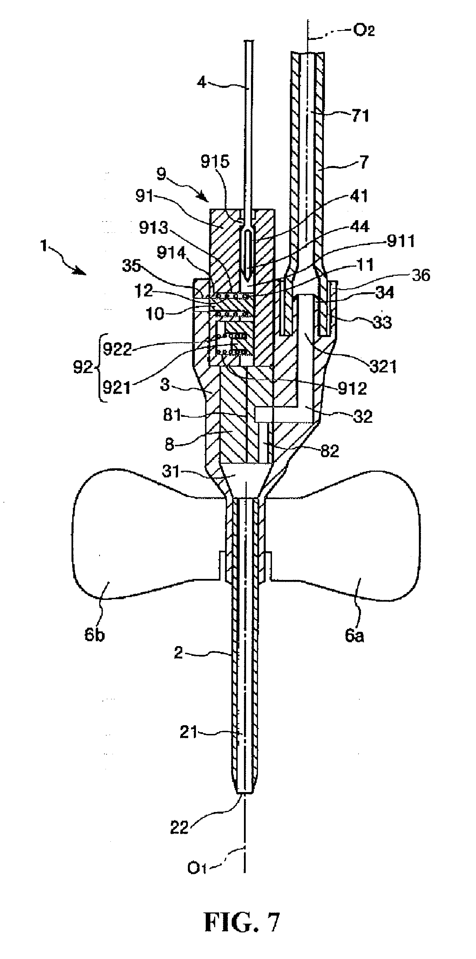

On the other hand, when the inner needle 4 is withdrawn from the inner needle passage 911, the fixing pin 10 is pushed by the coil spring 12 so as to move toward the right side in FIG. 7, while the left end part of the fixing pin 10 comes out of the through-hole 35. As a result, fixation of the protector 9 to the outer needle hub 3 is cleared (see FIG. 7).

Thus, in the present embodiment, the fixing means for fixing the protector 9 to the outer needle hub 3 is composed mainly of the through-hole 913, the fixing pin 10, the coil spring 12, and the inner needle 4.

Further, as shown in FIG. 6, in this embodiment, the fixing means operates after operation of the shutter means 92. Specifically, fixation of the protector 9 to the outer needle hub 3 by the fixing means is maintained in a condition wherein the shutter means 92 has been operated. Such a configuration ensures that the shutter means 92 is operated assuredly, in a condition in which fixation of the protector 9 to the outer needle hub 3 has been cleared. Therefore, an accident in which a worker erroneously punctures his or her finger or the like with the needlepoint 41, when discarding the inner needle 4 or on similar occasions, can be reliably prevented.

### <Engaging means>

Next, the engaging means shall be described below.

A basal part of the protector body 91 is provided with a reduced diameter part 915 thereon where the inner needle passage 911 is reduced in diameter. The inner diameter of the reduced diameter part 915 is greater than the outer diameters of both the intermediate outer diameter section 4b and the minimum outer diameter section 4c of the inner needle 4, while being smaller than the outer diameter of the maximum outer diameter section 4a.

This ensures that, when the inner needle 4 is withdrawn from the outer needle 2, the minimum outer diameter section 4c, the second varied outer diameter section 43 and the intermediate outer diameter section 4b can all pass through the reduced diameter part 915. However, the first varied outer diameter section 42 cannot pass through the reduced diameter part 915 and therefore becomes engaged with the reduced diameter part 915 (see FIG. 7).

In other words, in this embodiment, the first varied outer diameter section 42 and the reduced diameter part 915 collectively constitute an engaging means for facilitating engagement between the inner needle 4 and the protector 9.

With the engaging means provided as described above, during a series of operations in which the inner needle 4 is withdrawn from the outer needle 2, it is possible to engage the inner needle 4 with the protector 9, and to release the protector 9 from the outer needle hub 3 (see FIGS. 7 and 8), so that the operations can be carried out quite easily. In addition, the inner needle 4 can be prevented from coming off from the protector 9, so that the protector 9 remains in a state of covering the needlepoint 41.

Further, since the first varied outer diameter section 42 and the reduced diameter part 915 are formed respectively in the inner needle 4 and the protector 9, the configuration is simple, and an increase in the number of component parts is not required, which contributes to reductions in size and diameter.

In the above-described needle-stent device 1, as shown in FIGS. 1 and 5, the center axis O₁ of the outer needle 2 and the center axis O₂ of the tube 7 at its tip part are substantially in parallel with each other, in an assembled condition in which the tube 7 is connected to the basal part of the outer needle hub 3. In other words, the tube 7 protrudes from the base of the outer needle hub 3 in the basal direction (proximal direction).

Here, if the tube 7 projects toward a lateral side of the outer needle hub 3, the outer needle hub 3 is pulled sideways by the tube 7 when puncturing is performed with the outer needle 2 and the inner needle 4, with the result that good balance is lost. In such a case, therefore, intended operations are difficult to carry out.

In addition, if the tube 7 projects toward the upper side of the outer needle hub 3, there is a possibility that the tube 7 will become heavily bent (or kinked) when the outer needle hub 3 is fixed to the patient, when the outer needle 2 is made to indwell within a patient's blood vessel or the like.

Further, if the tube 7 projects toward the lateral side or toward the upper side of the outer needle hub 3, it becomes necessary to grip the inner needle hub 5 while avoiding the tube 7, so as not to pinch the tube 7 when only the outer needle 2 is moved forward into the blood vessel after the outer needle 2 has entered into the blood vessel. Accordingly, the operation in this instance is troublesome.

On the other hand, in the needle-stent device 1 according to the present invention, the tube 7 projects in the basal direction of the outer needle hub 3 and is covered by the inner needle hub 5. Therefore, the above-mentioned inconveniences can be prevented from occurring, whereby excellent operability is ensured.

Next, an example of a method of using the needle-stent device 1 (in the case of puncturing a blood vessel) shall be described in detail below.
[1] The needle-stent device 1 is placed in the locked state (the assembled condition), and a connector mounted to an end of an infusion line is preliminarily connected to the connector 72, thereby to enable an infusion liquid to be supplied from the infusion line.
   Incidentally, in this instance, a predetermined portion of the tube 7 or the infusion line is clamped, for example, by a clamp (an example of a conduit opening/closing means), thereby preliminarily closing the inner cavity.
[2] Next, closure of the tube 7 or the infusion line by the clamp or the like is released, and the infusion liquid from the infusion line is introduced through the tube 7 into the outer needle hub 3.
   The infusion liquid, which is introduced into the outer needle hub 3, fills up the conduit 32, the conduit 82, and the space on the tip side relative to the seal member 8 of the inside 31 of the outer needle hub 3, and the infusion liquid is introduced into the inner cavity 21 of the outer needle 2. This results in priming of the inner cavity 21 of the outer needle 2 with the infusion liquid. In this case, a portion of the infusion liquid flows out through the tip opening 22 of the outer needle 2.
[3] When priming has been completed as described above, the tube 7 or the infusion line is preliminarily closed again by a clamp or the like. Then, the vanes 6a and 6b are closed by pinching with the fingers and, with the vanes 6a and 6b serving as a gripping part (handling part), the outer needle 2 and the inner needle 4, which are bound together integrally, are caused to puncture a patient's blood vessel (vein or artery). In this instance, as mentioned above, the needle-stent device 1 is in a locked state, i.e., the inner needle hub is locked to the outer needle hub by the locking means (the tab 40 and the flange 522). Thus, a problem wherein the inner needle 4 becomes pushed by the skin and retracted into the outer needle 2 is prevented or restrained. Therefore, the inner needle 4 and the outer needle 2, and particularly the inner needle 4, can puncture the blood vessel (skin) assuredly.
   When the blood vessel is accessed by the outer needle 2, blood flows back in the basal direction inside the inner cavity 21 of the outer needle 2 via the groove 44 of the inner needle 4, due to the pressure inside the blood vessel (blood pressure). Therefore, flashback of the blood can be confirmed at least at one part of the outer needle 2, the outer needle hub 3, the inner needle hub 5, and the tube 7, which have a property for permitting visual confirmation thereof.
   After confirmation, the outer needle 2 and the inner needle 4 are further advanced by a minute distance in the tip direction.
   In addition, when the blood vessel is punctured in this manner, priming of the inner cavity 21 of the outer needle 2 with the infusion liquid has been completed, so that erroneous penetration of bubbles into the blood vessel can be prevented assuredly, and extreme high safety is secured.
   Further, as mentioned above, in the needle-stent device 1 according to the present invention, the center axis O₁ of the outer needle 2 and the center axis O₂ of the tube 7 at the tip part thereof are substantially in parallel with each other, in the assembled condition in which the tube 7 is connected to the basal part of the outer needle hub 3. Therefore, when puncturing is performed with the outer needle 2 and the inner needle 4, the tube 7 does not form an obstacle, and excellent operability is ensured.
[4] When the blood vessel is accessed by the outer needle 2, the tab 40 is turned so as to become disengaged from the flange 522, i.e., the needle-stent device (locking means) is placed in the unlocked state. In this condition, either the outer needle 2 or the outer needle hub 3 is fixed by one hand, while the inner needle hub 5 is pulled in the basal direction by gripping it with the other hand, to thereby withdraw the inner needle 4 from the outer needle 2. In this instance, as mentioned above, the inner needle hub 5 is not in a state of being fixed to the outer needle hub 3, i.e., the inner needle hub 5 is in a free state in relation to the outer needle hub 3, so that the inner needle 4 (inner needle hub 5) can be easily withdrawn from the outer needle 2 (outer needle hub 3).
[5] When the inner needle 4 is further moved in the basal direction and the needlepoint 41 passes through the slit 81, the seal member 8, which has a self-closing property, closes the slit 81 under its own elastic force. This ensures that leakage of liquid through the slit 81 is obviated, whereby asepsis inside of the outer needle hub 3 and the infusion line is secured.
[6] When the inner needle 4 is further moved and the needlepoint 41 passes through the vicinity of the recessed part 912 of the inner needle passage 911, the shutter member 921 is moved toward the side of the inner needle passage 911 under pushing by the coil spring 922, whereupon the right surface of the shutter member 921 is brought into contact with a surface opposed to the recessed part 912 of the inner needle passage 911. Namely, the shutter means 92 is transferred from the first posture (see FIG. 5) to the second posture (see FIG. 6).
   When the shutter means 92 is thus placed in the second posture, the shutter member 921 closes the inner needle passage 911. Therefore, even if the needlepoint 41 tends to return again in the tip direction, the needlepoint 41 abuts against the shutter member 921 and cannot be returned.
[7] When the inner needle 4 is further moved in the basal direction and the needlepoint 41 passes through the vicinity of the through-hole 913 of the inner needle passage 911, the fixing pin 10 moves toward the side of the inner needle passage 911 under pushing by the coil spring 12, whereupon the right surface of the fixing pin 10 abuts against a surface opposed to the through-hole 913 of the inner needle passage 911. In this instance, the left end part of the fixing pin 10 comes off from the through-hole 35 of the outer needle hub 3. As a result, fixation of the protector 9 to the outer needle hub 3 is cleared (see FIG. 7).
   In a condition in which fixation of the protector 9 to the outer needle hub 3 has been cleared, the shutter means 92 operates reliably, and therefore, an accident in which a worker or the like erroneously punctures his or her finger with the needlepoint 41 when discarding the inner needle 4, or on a similar occasion, can be prevented more securely.
[8] When the inner needle 4 is further moved in the basal direction, the first varied outer diameter section 32 cannot pass through the reduced diameter part 915 and becomes engaged with the reduced diameter part 915 (i.e., the inner needle 4 engages with the protector 9).
   When the inner needle hub 5 is further pulled in the basal direction in this condition, the protector 9, which is engaged with the inner needle 4, is moved in the basal direction together with the inner needle 4, and becomes separated from the outer needle hub 3 (see FIGS. 8 and 9). In this instance, the connection member 20 prevents the protector 9 from coming off of the inner needle hub 5.
   Incidentally, also at the time of carrying out the series of operations described above in steps [5] to [9], the center axis **O₁** of the outer needle 2 and the center axis of the tube 7 on the tip side may be kept substantially in parallel with each other by the guides 523 of the inner needle hub 5, so that operations can be carried out smoothly and assuredly.
[9] Next, the tube 7, which is inserted in the inner needle hub 5, is detached through the gap 521 (see FIG. 10).
   After the inner needle 4 has been withdrawn from the outer needle 2, the inner needle 4 and the inner needle hub 5 become unusable and, therefore, are discarded.
   The needlepoint 41 of the inner needle 4 is covered with the protector 9. In particular, the needlepoint 41 cannot be moved toward the tip side beyond the shutter means 92 and thereby protrude from the tip of the protector 9. Therefore, an accident in which a worker or a similar person discarding the inner needle 4 erroneously punctures his or her finger with the needlepoint 41 can be prevented.
[10] Next, the tab 40 is turned to the stored state. This ensures that the tab 40 will not obstruct the operation of fixing the outer needle hub 3 to the skin by use of a pressure sensitive adhesive tape or the like.

In the above stored state, the vanes 6a and 6b are opened, and the outer needle hub 3 is fixed onto the skin by a pressure sensitive adhesive tape or the like. Also, obstruction of the tube 7 or the infusion line by the clamp is released, whereby the infusion is started.

The infusion supplied via the infusion line is injected into the blood vessel of a patient after the infusion passes through the lumens of the connecter 72, the tube 7, the outer needle hub 3, and the outer needle 2.

### <Second Embodiment>

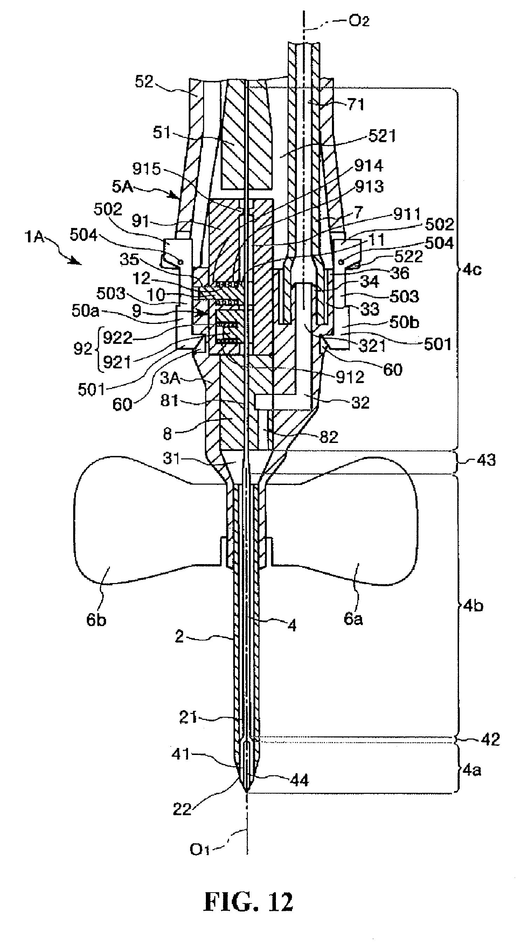

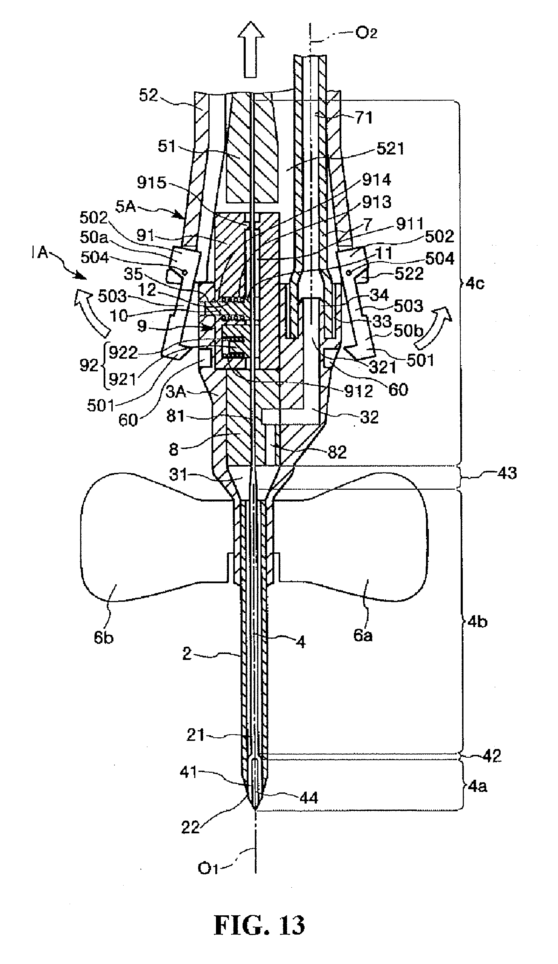

FIGS. 12 and 13 are partial longitudinal sectional views of a needle-stent device according to the present invention (second embodiment). Incidentally, in the following descriptions, the upper side in FIGS. 12 and 13 will be referred to as "the base", whereas the lower side will be referred to as "the tip".

The second embodiment of the needle-stent device according to the present invention will be described below referring to the figures. The following descriptions shall be centered on differences between this embodiment and the above-described embodiment, and descriptions of the same items previously discussed above shall be omitted.

The present embodiment is the same as the first embodiment above, except for the configuration of the locking means.

As shown in FIGS. 12 and 13, an inner needle hub 5A is provided with a pair of clamp pieces 50a and 50b. The clamp pieces 50a and 50b are disposed opposite to each other, with the center axis of the inner needle hub 5A residing therebetween. Since the clamp pieces 50a and 50b have substantially the same configuration (shape), the clamp piece 50a, and a recessed part (engaging part) 60 that engages therewith, will be described representatively below.

The clamp piece 50a includes a plate-like clamp piece body 503, a claw part (projecting part) 501 provided on the tip side of the clamp piece body 503, a claw part operating member 502 provided on the base side of the clamp piece body 503, and a turning shaft 504.

The claw part 501 is formed so as to project from a surface on the inner side (on the right side in FIG. 12, as well as in FIG. 13) of the clamp piece body 503. The claw part 501 has a wedge-like shape.

The claw part operating member 502 is formed so as to project from a surface on the outer side (on the left side in FIG. 12, as well as in FIG. 13) of the clamp piece body 503. By operating the claw part operating member 502, namely, by pressing the claw part operating member 502 to the right side in FIG. 12, the claw part 501 can easily be turned clockwise around the turning shaft 504.

In addition, the turning shaft 504 forms a shaft by which the clamp piece body 503 is rotatably supported, so as to be turnable relative to the inner needle hub 5A.

The recessed part 60 is provided on an outer peripheral portion of the outer needle hub 3A, corresponding to the claw part 501. In this embodiment, two recessed parts 60 are provided.

As shown in FIG. 12, in the assembled condition, the claw parts 501 of the clamp piece 50a and the clamp piece 50b are engaged with the recessed parts 60, respectively. This places the needle-stent device 1A in a locked state, in which the inner needle hub 5A is locked to the outer needle hub 3A.

Moreover, as shown in FIG. 13, by operating the claw part operating members 502 of the clamp piece 50a and the clamp piece 50b while the clamp pieces 50a, 50b are in a locked state (engaged state), specifically, by moving the claw part operating members 502 toward each other, the claw parts 501 are spaced away from each other and the claw parts 501 become disengaged from the recessed parts 60. As a result, the needle-stent device 1A assumes an unlocked state in which the locked state thereof is released.

When a puncturing operation is conducted with the needle-stent device 1A in the locked state by pinching the vanes 6a and 6b with the fingertips, the inner needle hub 5A is in a state of being fixed to the outer needle hub 3A. Therefore, a problem in which the inner needle 4 may be pushed by the skin and retracted into the outer needle 2 before the skin (blood vessel) is punctured can be prevented or restrained, so that the inner needle 4 punctures the skin assuredly. In other words, the needle-stent device 1A is excellent in operability at the time the puncturing operation is carried out.

In addition, by operating the claw part operating members 502 after puncturing, an unlocked state can be obtained and, hence, the inner needle 4 can be withdrawn from the outer needle 2 through an easy operation. Then, the outer needle 2, from which the inner needle 4 has been pulled off, can be left to indwell inside the blood vessel.

Thus, in the needle-stent device 1A, the clamp piece 50a (claw part 501), the clamp piece 50b (claw part 501), and the recessed parts 60 collectively constitute a locking means for fixing the inner needle hub 5A to the outer needle hub 3A in the assembled condition.

In the needle-stent device 1A, when the inner needle 4 is withdrawn from the outer needle 2, the inner needle hub 5A provided with the inner needle 4 thereon is gripped to withdraw the inner needle 4. In view of this, the inner needle hub 5A preferably is provided with the clamp pieces 50a and 50b. In other words, when the inner needle hub 5A is provided with the clamp pieces 50a and 50b, operability of the needle-stent device 1A is enhanced.

Incidentally, the present embodiment is not limited by the configuration wherein the clamp pieces 50a and 50b are provided on the side of the inner needle hub 5A. The clamp pieces 50a and 50b may also be provided on the side of the outer needle hub 3A. In the event that the clamp pieces 50a and 50b are provided on the side of the outer needle hub 3A, the recessed parts 60 that engage with the claw parts 501 must be provided on the side of the inner needle hub 5A.

In addition, the present embodiment is not limited by the configuration in which two (a pair of) clamp pieces are provided opposite to each other. For example, three or more clamp pieces may be provided along the outer periphery of the inner needle hub 5A.

### <Third Embodiment>

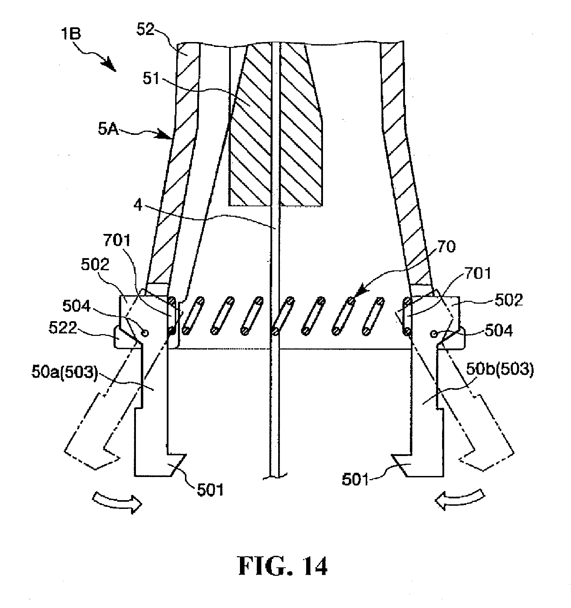

FIG. 14 is a partial longitudinal sectional view of an inner needle hub possessed by a needle-stent device according to the present invention (third embodiment). Incidentally, in the following description, the upper side in FIG. 14 will be referred to as "the base", whereas the lower side will be referred to as "the tip".

The third embodiment of the needle-stent device according to the present invention will be described below with reference to the figure. The following descriptions will be centered on differences between the present embodiment and the above-described embodiments, wherein descriptions of the same items as those already discussed above shall be omitted.

The present embodiment is the same as the second embodiment above, except for the configuration of the locking means.

As shown in FIG. 14, a compression coil spring (urging part) 70 is disposed (interposed) between two claw part operating members 502. The compression coil spring 70 is in contact at each end part 701 thereof with the claw part operating members 502.

The compression coil spring 70 disposed in this manner acts to urge the claw part operating members 502 in a direction (the direction of the arrow in FIG. 14) such that the claw parts 501 move toward each other. This ensures that the needle-stent device 1B is reliably placed in a locked state when in an assembled condition. In other words, it is possible to securely prevent the needle-stent device 1B from being unintentionally placed in an unlocked state, with the result that the inner needle hub 5A comes off from the outer needle hub 3A.

Thus, in the needle-stent device 1B, a clamp piece 50a (claw part 501), a clamp piece 50b (claw part 501), recessed parts 60, and the compression coil spring 70 collectively constitute a locking means for fixing the inner needle hub 5A to the outer needle hub 3A in the assembled condition.

While the needle-stent device according to the present invention has been described with reference to various embodiments shown in the drawings, the invention is not limited to these embodiments. Various components or parts of the needle-stent device can be replaced by other components having arbitrary configurations, which can exhibit functions that are the same or equivalent to the above-mentioned functions. Further, arbitrary structures may be added to the above-described embodiments.

In addition, the needle-stent device according to the present invention is not limited to one that is used in a condition of being inserted into a blood vessel. The needle-stent device may also be used in a condition of being inserted, for example, into the abdominal cavity, the thoracic cavity, a lymph vessel, the vertebral canal, or the like.

The shape of the slit in the seal member is not limited to a straight line segment, and the slit may have, for example, a cross (+) shape, a Y shape, a T shape, or an H shape.

Further, the seal member is not limited to being provided with a slit therein which is closed in the natural condition. Alternatively, the seal member may be provided with a hole, for example. In this case, preferably, the seal member is disposed inside the outer needle hub in a state of being compressed, to such an extent that the hole is closed.

In addition, the locking means is not limited to the configuration shown in the drawings. For example, the locking means may be composed of a fitting disposed between a tip part of the inner needle hub and a basal part of the outer needle hub. In the case that the locking means is composed of such a fitting, the needle-stent device, when in a locked state (fitted state), is placed in an unlocked state by pulling the inner needle hub.

In addition, a cap may be provided, which is mounted to the basal part of the outer needle hub after the inner needle has been withdrawn from the outer needle. This ensures that leakage of liquid through the base of the outer needle hub can be prevented more securely. The cap may be formed as a unitary body together with the outer needle hub, or the cap may be provided separately from the outer needle hub. In addition, the method of fixing the cap to the outer needle hub may be any method, such as a method based on friction, hooking, etc.

In addition, the protector is not limited to the configuration shown in the figures. For example, the protector may be provided so as to be turnable (displaceable) between a position where the protector covers at least the needlepoint of the inner needle, and another position at which the protector is spaced from the inner needle.

Further, the connector provided at the end part of the tube is not particularly limited. Examples of usable connectors include a needleless connector, as described in Japanese Laid-Open Patent Publication No. 2005-261931, a three-way cock, etc.

In addition, the component provided at the end part of the tube is not limited to the above-mentioned connector. A cap, an air filter or the like may also be adopted, for example.

Moreover, in the needle-stent device according to the present invention, the connector, the cap, and the air filter may be attached in a switchable manner, as required, to the end part of the tube.

### INDUSTRIAL APPLICABILITY

The needle-stent device according to the present invention includes an inner needle having a sharp needlepoint at a tip thereof, an inner needle hub fixed to a basal part of the inner needle, a hollow outer needle into which the inner needle is inserted, an outer needle hub fixed to a basal part of the outer needle, a tube connected to a basal part of the outer needle hub so that an inner cavity thereof communicates with an inner cavity of the outer needle, an opening formed at a basal part or a side part of the outer needle hub so as to communicate with the inner cavity of the outer needle, a handling part, which is provided in the outer needle hub and by which the inner needle and the outer needle are moved in a longitudinal direction thereof in an inserted condition in which the inner needle is inserted into the outer needle, and a locking means which is switchable, in the inserted condition, between a locked state in which the inner needle hub is locked to the outer needle hub, and an unlocked state in which the locked state is released. Therefore, at the time of performing a puncturing operation, a locked state is attained, in which the inner needle hub is locked to the outer needle hub, so that the inner hub can be fixed securely to the outer needle hub, and therefore movement of the inner needle can be inhibited or suppressed reliably. As a result, the inner needle is capable of puncturing the skin without fail. In addition, since the unlocked state, wherein the locked state is released, is obtained after puncturing has been completed, the inner needle can easily be pulled out of the outer needle. In other words, according to the present invention, excellent operability can be obtained when carrying out a puncturing operation, and a line for an infusion liquid or the like can be secured easily and assuredly. Accordingly, the needle-stent device of the present invention has industrial applicability.

## Claims

1. A needle-stent device comprising:
an inner needle having a sharp needlepoint at a tip thereof;
an inner needle hub fixed to a basal part of said inner needle;
a hollow outer needle into which said inner needle is inserted;
an outer needle hub fixed to a basal part of said outer needle;
a tube connected to a basal part of said outer needle hub so that an inner cavity thereof communicates with an inner cavity of said outer needle;
an opening formed at a basal part or a side part of said outer needle hub so as to communicate with the inner cavity of said outer needle;
a handling part, which is provided in said outer needle hub and by which said inner needle and said outer needle are moved in a longitudinal direction thereof in an inserted condition in which said inner needle is inserted in said outer needle; and
a locking means which is switchable, in said inserted condition, between a locked state in which said inner needle hub is locked to said outer needle hub, and an unlocked state in which said locked state is released.

2. The needle-stent device as set forth in claim 1, wherein a tube is connected to said opening.

3. The needle-stent device as set forth in claim 2, wherein said tube is inserted in said inner needle hub.

4. The needle-stent device as set forth in claim 1,
wherein said locking means has a projecting part disposed on one of said inner needle hub and said outer needle hub, and an engaging part disposed on the other of said inner needle hub and said outer needle hub, which is capable of engagement with said projecting part, and
wherein said engaging part engages with said projecting part in said locked state, while said engaging part is disengaged from said projecting part in said unlocked state.

5. The needle-stent device as set forth in claim 4, wherein said engaging part is rotatably supported, and is turned between said locked state and said unlocked state.

6. The needle-stent device as set forth in claim 4 or 5, wherein said engaging part is disposed on said outer needle hub and serves as said handling part in said unlocked state.

7. The needle-stent device as set forth in claim 4 or 5, wherein said engaging part is disposed on said outer needle hub, and is switchable not only between said locked state and said unlocked state, but also is switchable to a retracted state, in which at least a portion of said engaging part is retracted from an outer peripheral portion of said outer needle hub.

8. The needle-stent device as set forth in claim 7, wherein said retracted state is a stored state in which at least a portion of said engaging part is stored in said outer needle hub.

9. The needle-stent device as set forth in claim 4, wherein said projecting part comprises a plurality of claw parts provided along the outer periphery of said inner needle hub, and wherein a plurality of said engaging parts are provided corresponding respectively to said claw parts.

10. The needle-stent device as set forth in claim 9, further comprising a claw part operating member by which said claw parts are moved toward and away from each other.

11. The needle-stent device as set forth in claim 10, wherein respective claw part operating members are provided at each of said claw parts, and said claw parts are spaced away from each other when said claw part operating members are moved toward each other.

12. The needle-stent device as set forth in claim 10 or 11, further comprising an urging part for urging said claw part operating member in a direction such that said claw parts are moved toward each other.

13. The needle-stent device as set forth in claim 9, wherein said claw part is provided on said inner needle hub, and said engaging part is provided on said outer needle hub.
